# EUROPÄISCHE PATENTSCHRIFT

(11) **EP 3 500 149 B1**
(45) Veröffentlichungstag und Bekanntmachung des Hinweises auf die Patenterteilung: **29.04.2020**
(21) Anmeldenummer: 17822552.0
(22) Anmeldetag: 22.11.2017
(51) Int. Cl.: A61B 1/00, G02B 23/24, H02K 41/02, G02B 7/08

(54) **ELEKTROMAGNETISCHER AKTUATOR FÜR EIN CHIRURGISCHES INSTRUMENT**
ELECTROMAGNETIC ACTUATOR FOR A SURGICAL INSTRUMENT
ACTIONNEUR ÉLECTROMAGNÉTIQUE POUR INSTRUMENT CHIRURGICAL

(30) Priorität: 29.11.2016 DE 102016122951
(43) Veröffentlichungstag der Anmeldung: 26.06.2019
(73) Patentinhaber: Olympus Winter & Ibe GmbH, 22045 Hamburg (DE)
(72) Erfinder: WIETERS, Martin, 22885 Barsbüttel (DE)
(74) Vertreter: Meissner Bolte Partnerschaft mbB
(86) Internationale Anmeldenummer: PCT/EP2017/080060
(87) Internationale Veröffentlichungsnummer: WO 2018/099783

(56) Entgegenhaltungen:
- DE-A1-102012 104 832
- DE-A1-102012 219 354
- US-A1- 2011 210 690
- US-A1- 2015 282 692
- US-B1- 7 859 144

## Beschreibung

Die Erfindung betrifft einen elektromagnetischen Aktuator für ein chirurgisches oder medizinisches Instrument gemäß dem Oberbegriff des Anspruchs 1.

Chirurgische oder medizinische Instrumente wie beispielsweise Endoskope mit einem länglichen Schaft können im Selbigen ein distal angeordnetes Objektiv aufweisen. Dieses Objektiv dient der Betrachtung eines Gegenstandes im Inneren eines Körpers. Zur Betrachtung dieses Gegenstandes durch eine Bedienperson wird das durch das Objektiv erzeugte Bild mittels eines Bildleiters oder elektronisch über eine Kamera durch den Schaft nach außen geführt. Das Objektiv kann dabei ein optisches Element wie eine Linse oder eine Gruppe von Linsen aufweisen, welche in Richtung einer optischen Achse des Objektivs zur Fokussierung und/oder zur Veränderung der Brennweite durch einen Aktuator verschiebbar gelagert ist. Dieser elektromagnetisch antreibbare Aktuator ist in dem Schaft des Endoskopes angeordnet und daher nur wenige Millimeter groß.

Ein wesentlicher Bestandteil des Aktuators ist ein zylindrischer Stator, in dem ein Element angeordnet ist, welches entlang der optischen Achse bewegbar ist. Der Stator weist mindestens zwei ringartige, axial magnetisierte Permanentmagnete sowie mindestens zwei Spulen zur Erzeugung eines elektromagnetischen Feldes auf. Das bewegliche Element ist als ein in einem Gleitrohr angeordneter Läufer ausgebildet. Das Gleitrohr wird dabei durch die ringartigen Permanentmagnete und die Spulen umschlossen.

Der bewegliche Läufer in dem Gleitrohr dient der Aufnahme des Objektivs, insbesondere der Linse oder der Linsengruppe, und besteht aus einem magnetisierbaren Material. Das Gleitrohr weist an seinen Enden jeweils Anschläge auf, welche die axiale Bewegung des Läufers in dem Gleitrohr begrenzt. Durch die Anordnung der Permanentmagnete um das Gleitrohr befindet sich der Läufer an den Enden des Gleitrohres jeweils in einer bistabilen Lage. Durch die Bestromung der Spulen wird der Läufer durch das induzierte Magnetfeld der Spulen aus einer Endlage in die andere Endlage bewegt. Wenn kein magnetisches Feld durch die Spule induziert wird, verbleibt der Läufer samt Linsen in einer seiner Endstellungen.

Da der zur Verfügung stehende Raum in dem Schaft eines Endoskops sehr begrenzt ist, muss der Aktuator sowie die Linsen entsprechend miniaturisiert werden. Das stellt nicht nur besonders hohe Anforderungen an die Genauigkeit der Spulen und des Läufers sowie der Anordnung der Linsen dar, sondern auch an die Form der Permanentmagnete. Bereits kleinste, herstellungsbedingte Variationen in der Dimensionierung der Permanentmagnete wirken sich negativ auf das Feldlinienbild der Magnete und somit auf das Schaltverhalten des Läufers aus. Eine Konsequenz eines leicht zu großen oder zu kleinen Permanentmagneten kann darin bestehen, dass die Endlagen des Läufers nicht mehr bistabil sind und sich somit das Objektiv nicht mehr einwandfrei fokussieren lässt.

Elektromagnetische Aktuatoren gemäß des Oberbegriffs des Anspruchs 1 sind aus Dokumenten US 2011/210690 A1 und US 7,859,144 B1 bekannt.

Die Aufgabe der Erfindung besteht somit darin, einen elektromagnetischen Aktuator für ein chirurgisches oder medizinisches Instrument zu schaffen, der sich besonders zuverlässig schalten lässt.

Ein Aktuator zur Lösung dieser Aufgabe weist die Merkmale des Anspruchs 1 auf. Demnach ist es vorgesehen, dass die Permanentmagnete derart um das Gleitrohr angeordnet sind, dass ein magnetischer Südpol des einen Permanentmagneten einem magnetischen Nordpol des anderen Permanentmagneten zugewandt ist. Durch diese Gleichpolung der Permanentmagnete überlagern sich die beiden magnetischen Felder wenigstens teilweise und wirken so zusammen. Durch diese Gleichorientierung der magnetischen Feldvektoren wirken sich geringfügige Abweichungen der Dimensionierung der Magnete weniger stark auf das Magnetfeld aus, als wenn die Feldlinien entgegengesetzt ausgerichtet wären. Somit gestattet diese gleiche Polung der Permanentmagnete eine höhere Toleranz in der Herstellung der Permanentmagnete. Da somit geringfügige Unterschiede in Länge und/oder Durchmesser der Permanentmagnete durch die Wechselwirkung der beiden Permanentmagnete ausgeglichen werden können, gestaltet sich auch die Positionierung des Läufers in den beiden Endlagen im Gleitrohr als besonders stabil. Auf diese Weise lässt sich ein besonders zuverlässiges Schalten des Aktuators, insbesondere des Läufers, realisieren.

Ein weiteres bevorzugtes Ausführungsbeispiel der vorliegenden Erfindung kann es vorsehen, dass die beiden Spulen eine entgegengesetzte Wickelrichtung aufweisen. Durch diese entgegengesetzte Wickelrichtung sind die magnetischen Feldlinien des durch einen Strom gleichen Vorzeichens erzeugten magnetischen Feldes in den Spulen entgegengerichtet. Wenn allerdings nur eine Spule zur Zeit durch einen Strom gleichen Vorzeichens bestromt wird, werden magnetische Felder induziert, deren Feldvektoren abwechselnd verschiedene Orientierungen entlang der optischen Achse aufweisen. Somit lässt sich durch entsprechende Schaltung der Spulen der Läufer aus einer stabilen Endlage in dem Gleitrohr in die jeweils andere stabile Endlage bewegen. Maßgeblich dabei ist, dass die durch die Spule induzierte magnetische Kraft auf den Läufer zumindest kurzzeitig größer ist, als die eines Permanentmagnetens. Durch diese Anordnung der beiden Spulen bzw. durch deren entgegengesetzte Wicklung, ist eine besonders einfache Kontaktierung der Spulen mit kurzen Zuleitungen realisierbar.

Insbesondere ist erfindungsgemäß vorgesehen, dass jeder Spule eine Diode vorgeschaltet ist, die unterschiedlich orientiert sind, sodass die Durchlassrichtungen der Dioden bezüglich der Spulen umgedreht sind. Je nach Bestromung der Spulen wird somit die eine oder die andere Spule in Abhängigkeit von der Durchlassrichtung der Diode angesteuert, wodurch jeweils nur in einer Spule ein magnetisches Feld induziert wird. Durch die Anordnung der Dioden wird ein Schaltkreis geschaffen, der mit möglichst wenig elektrischen Leitungen auskommt. Da insbesondere der Platz in dem Schaft sehr begrenzt ist, stellt dies einen besonderen Vorteil der vorliegenden Erfindung dar. Da der Schaft in seinem Inneren einen hermetisch dichten Raum darstellt, wirkt sich die Reduzierung der notwendigen elektrischen Leitungen besonders vorteilhaft aus, da somit weniger Leitungen zu berücksichtigen sind, die eine hermetische Abdichtung ansonsten erschweren könnten.

Weiter ist erfindungsgemäß vorgesehen, dass die Spulen separat ansteuerbar sind, wobei die Spulen mit einem Wechselimpuls elektromagnetischer Energie betreibbar sind. Diese Impulse sind nur wenige, insbesondere zehn bis zwanzig Millisekunden lang und wechseln periodisch ihr Vorzeichen. Darüber hinaus kann es erfindungsgemäß vorgesehen sein, dass die Spulen zwischen den Permanentmagneten auf dem Gleitrohr angeordnet sind, wobei die Permanentmagnete Endbereichen des Gleitrohres zugeordnet sind. Durch diese Anordnung der Spulen zwischen den Permanentmagneten koppeln die Magnetfelder insbesondere die induzierten Magnetfelder, besonders wirksam zusammen, sodass der Läufer einerseits in seinen stabilen Endlagen verweilt und andererseits auf eine verlässliche Art und Weise aus der einen Lage in die andere Lage bewegbar ist.

Ein besonders vorteilhaftes Ausführungsbeispiel der vorliegenden Erfindung kann es außerdem vorsehen, dass zwischen den Permanentmagneten ein zylindrischer Rückschluss um die Spulen angeordnet ist, insbesondere dieser Rückschluss Polschuhe aufweist, die zwischen den Spulen und/oder zwischen den Permanentmagneten und den Spulen angeordnet sind. Durch diese Polschuhe lässt sich die magnetische Felddichte an einigen Stellen derart umleiten bzw. konzentrieren, dass der Schaltprozess des Läufers sogar noch verbessert wird. Insbesondere die Anordnung eines Polschuhs zwischen den Spulen wirkt sich als besonders vorteilhaft für ein störungsfreies Schalten zwischen den beiden Endpositionen des Läufers aus.

Weiter kann es die Erfindung vorsehen, dass die Spulen derart bestrombar sind, dass sich in dem Rückschluss ein magnetischer Fluss ausbildet mit der gleichen Orientierung wie der magnetische Fluss der Permanentmagnete. Der Rückfluss bezweckt somit, dass sich zumindest während der Bestromung der Spulen ein homogenes magnetisches Feld um das Gleitrohr, bzw. in dem Gleitrohr ausbildet.

Besonders bevorzugt kann es die vorliegende Erfindung weiter vorsehen, dass den Endbereichen des Gleitrohres jeweils ein Anschlag zugeordnet ist, um den Hub des beweglichen Läufers zu begrenzen. Diese Anschläge können innerhalb oder außerhalb des Gleitrohres angeordnet sein und definieren die Länge, über die sich das Objektiv bzw. die Linsengruppe fokussieren lässt.

Insbesondere kann es die Erfindung außerdem vorsehen, dass der Läufer als Hohlzylinder aus einem weichmagnetischen Material, vorzugsweise zur Aufnahme mindestens einer Linse, ausgebildet ist. Je geringer der Energieaufwand zur Magnetisierung des Läufers ist, desto weniger magnetische Energie muss von den Spulen aufgebracht bzw. von den Permanentmagneten zur Schaltung des Läufers erzeugt werden. Als besonders geeignet haben sich Stahllegierungen erwiesen.

Eine weitere vorteilhafte Ausbildung der vorliegenden Erfindung kann darin gesehen werden, dass der Läufer zwei Polschuhe aufweist, die den Endbereichen des Läufers zugeordnet sind. Durch die Ausbildung von Polschuhen koppelt dieser besonders bevorzugt an die Magnetfelder an, so dass ein schneller und verlässlicher Schaltprozess des Aktuators möglich ist.

Ein bevorzugtes Ausführungsbeispiel der Erfindung wird nachfolgend anhand der Zeichnung näher erläutert. In dieser zeigen:
Fig. 1 eine schematische Ansicht eines Endoskops,
Fig. 2 ein Schnitt durch einen schematisiert dargestellten Aktuator in einer ersten Stellung,
Fig. 3 ein Schnitt durch den Aktuator in einer zweiten Stellung,
Fig. 4 ein Schnitt durch ein weiteres Ausführungsbeispiel eines Aktuators, und
Fig. 5 ein Schaltbild einer elektrischen Ansteuerung eines Spulenpaares.

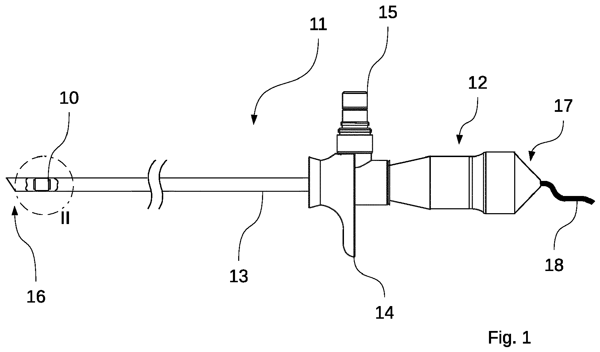

Die vorliegende Erfindung ist gerichtet auf einen Aktuator 10 für ein chirurgisches oder medizinisches Instrument. Bei diesem chirurgischen oder medizinischen Instrument kann es sich beispielsweise um ein Endoskop 11 handeln. Allerdings sei ausdrücklich darauf hingewiesen, dass der hier beschriebene Aktuator 10 auch in anderen Instrumenten einsetzbar ist und mit dem in der Fig. 1 dargestelltem Endoskop 11 lediglich eine Einsetzmöglichkeit des Aktuators 10 beispielhaft dargestellt werden soll.

Das in der Fig. 1 dargestellte Endoskop 11 weist proximal einen Hauptkörper 12 auf mit einem sich daran distal anschließenden langgestreckten, rohrförmigen Schaft 13. Der Anschauung halber ist dieser Schacht 13 verkürzt dargestellt. Der Hauptkörper 12 weist außerdem einen Griff 14 auf, mit dem eine Bedienperson das Endoskop 11 greifen und bedienen kann. Des Weiteren ist dem Hauptkörper 12 ein Anschluss 15 zugeordnet, über den beispielsweise Licht über eine Faseroptik durch den Schaft 13 dem distalen Ende 16 des Endoskops 11 zuführbar ist. Das proximale Ende 17 des Endoskops 10 ist über einen Leiter 18 mit einer Energieversorgung und einer Steuereinheit verbindbar.

In dem Schaft 13 des Endoskops 11 ist eine Optik installiert, die es ermöglicht das Innere eines zu operierenden nicht dargestellten Körpers abzubilden. Dazu wird beispielsweise über eine nicht dargestellte Sensoreinheit, die sich in dem distalen Ende 16 des Endoskops befindet, ein Bild aufgenommen und über den Leiter 18 einer externen Bildwiedergabeeinrichtung, wie beispielsweise einem Monitor, zugeführt. Es ist jedoch auch denkbar, dass das Bild über optische Fasern oder Stablinsen den proximalen Ende 17 des Endoskops 10 zugeführt wird.

Um das Objekt im Inneren des Körpers scharf darzustellen, ist dem distalen Ende 16 im Inneren des Schaftes 13 ein Objektiv bzw. mindesten eine Linse zugeordnet, die zum Fokussieren sowie zum Einstellen der Brennweite beweglich gelagert ist. Diese bewegliche Lagerung der nicht dargestellten Linse wird durch einen elektromagnetischen Aktuator 10 im Inneren des Schaftes 13 realisiert.

Bei dem in der Fig. 1 dargestellten Ausführungsbeispiel eines Endoskops ist für Anschauungszwecke der Schaft 13 am distalen Ende 16 "aufgeschnitten" dargestellt, sodass der Blick frei ist auf den Aktuator 10. Die mindestens eine Linse ist im Inneren dieses Aktuators 10 angeordnet. Elektrische Leitungen für die Energieversorgung sowie zur Ansteuerung des Aktuators verlaufen im Inneren des Schaftes 13 zum proximalen Ende 17 des Endoskops 11 und werden beispielsweise durch den Leiter 18 weiteren nicht dargestellten Steuereinheiten zugeführt.

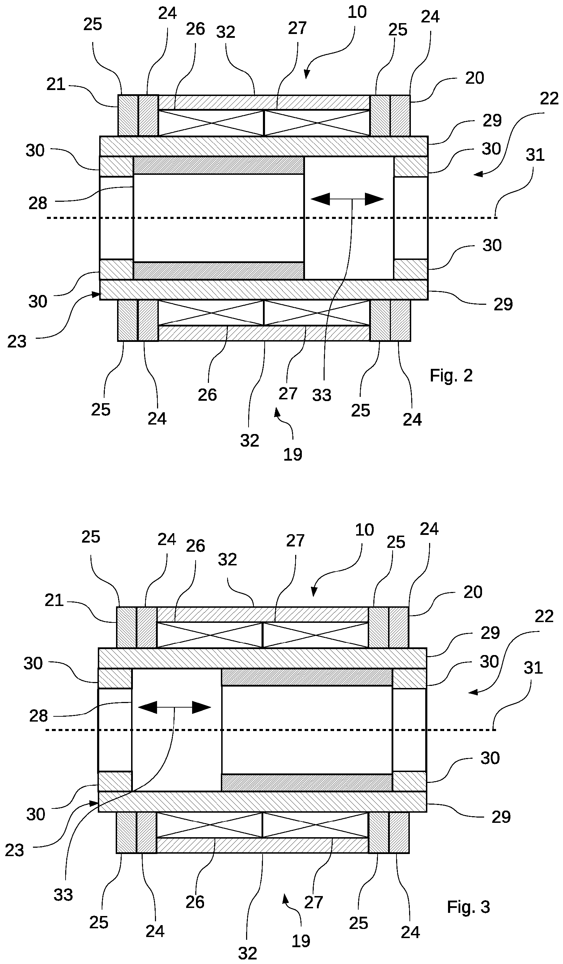

Der elektromagnetische Aktuator 10 besteht im Wesentlichen aus einem zylindrischen Stator 19 und einem in dem Stator 19 angeordneten beweglichen Element (Fig. 2). Um diesen zylindrischen Stator 19 sind ringartig zwei Permanentmagnete 20, 21 angeordnet. Diese Permanentmagnete 20, 21 sind den Endbereichen 22, 23 des Stators 19 zugeordnet. Erfindungsgemäß sind die beiden Permanentmagnete 20, 21 derart zueinander orientiert, dass sich beispielsweise ein Nordpol 24 des Permanentmagneten 22 gegenüber einem Südpol 25 des Permanentmagneten 21 befindet. Es ist jedoch genauso denkbar, dass sich der Südpol 25 des Permanentmagneten 20 und der Nordpol 24 des Permanentmagneten 21 gegenüber liegen. Durch diese Gleichpolung der beiden Permanentmagnete 20, 21 arbeiten diese derart zusammen, dass sich ein besonders homogenes Magnetfeld ausbildet.

Bei dem in der Fig. 2 dargestellten Ausführungsbeispiel des Aktuators 10 befinden sich zwischen den Permanentmagneten 20, 21 auf dem zylindrischen Stator 19 zwei Spulen 26, 27. Diese Spulen 26, 27 sind ebenfalls ringartig um den Stator 19 positioniert. Erfindungsgemäß ist die Wicklung der Spulen 26, 27 genau entgegengesetzt zueinander, sodass beim Anlegen eines elektrischen Stroms an die Spulen 26, 27 zwei entgegengesetzte magnetische Felder induziert werden.

Das bewegliche Element in Inneren des Stators 19 wird durch einen Läufer 28 gebildet. Bei diesem Läufer handelt es sich um einen Hohlzylinder aus einem weichmagnetisierbaren Material. Bei der Anwendung des Aktuators 10 in einem Endoskop 11 wird innerhalb dieses Läufers 28 das Objektiv bzw. die mindestens eine Linse positioniert.

Der Läufer 28 ist innerhalb eines Gleitrohres 29 beweglich gelagert. Um dieses Gleitrohr 29 sind die zuvor beschriebenen Permanentmagnete 20, 21 und die beiden Spulen 26, 27 ringartig angeordnet. Um die Bewegung des Läufers 28 in dem Gleitrohr 29 zu begrenzen, sind den Endbereichen 22, 23 jeweils Anschläge 30 zugeordnet. Diese Anschläge 30 können ebenfalls ringartig oder einfach nur als quaderförmige Stopper ausgebildet sein, um den Läufer 28 daran zu hindern, das Gleitrohr 29 zu verlassen.

Der Läufer 28 ist derart in dem Gleitrohr 29 beweglich gelagert, dass sich dieser parallel zu einer optischen Achse 31 verschieben lässt. Diese optische Achse 31 entspricht auch der optischen Achse der mindestens einen Linse. Sämtliche Elemente des Aktuators 10 wie der Stator 19, die Permanentmagnete 20, 21 sowie die Spulen 26, 27 sind symmetrisch um diese optische Achse 31 angeordnet.

Um das durch die Spulen 26, 27 und die Permanentmagnete 20, 21 generierte magnetische Feld zur optischen Achse 31 hin zu führen, bzw. zu verstärken, ist bei dem in der Fig. 2 dargestelltem Ausführungsbeispiel der vorliegenden Erfindung um die Spulen 26, 27 ein ebenfalls ringartiger metallischer Rückschluss 32 angeordnet. Für den Fall, dass keine der Spulen 26, 27 bestromt wird, befindet sich der Läufer 28 angezogen durch den Permanentmagnet 20 oder 21 in einer der beiden Endlagen. In dieser Lage wird der Läufer 28 durch einen der beiden Permanentmagnete 20, 21 gerade in dieser bistabilen Lage fixiert. Das heißt ohne Wirkung einer weiteren Kraft wird der Läufer 28 diese Endlage nicht verlassen. Zum Fokussieren der Linse bzw. zum Einstellen deren Brennweite muss der Läufer 28 entlang der optischen Achse 31 bewegt werden. Dazu wird eine oder beide Spulen 26, 27 bestromt, wodurch der Läufer 28 bedingt durch die induzierte magnetische Kraft entlang der Pfeilrichtung 33 in die zweite bistabile Lage transportiert. Wenn keine weiteren Kräfte auf den Läufer 28 in dieser bistabilen Lage wirken, verweilt er in dieser Endstellung (Fig. 3).

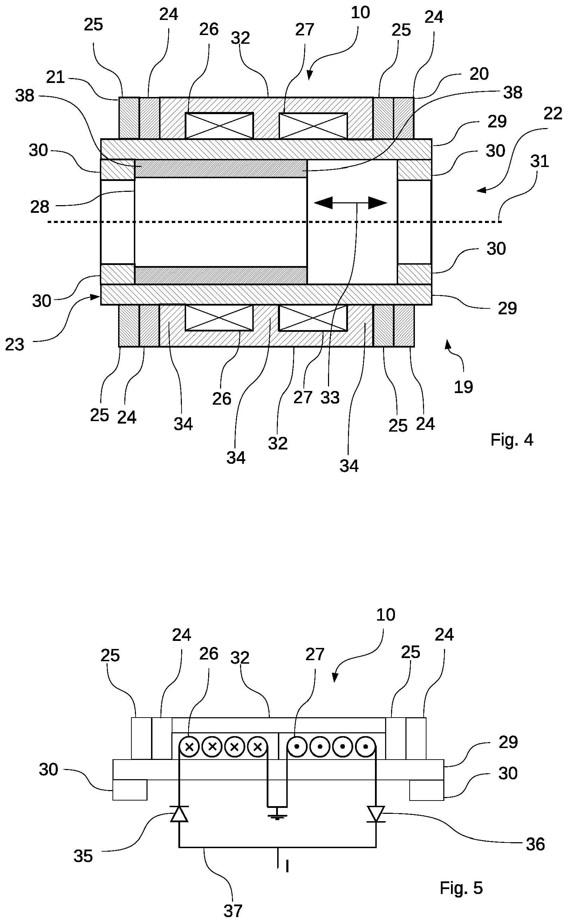

Ein weiteres Ausführungsbeispiel des Aktuators 10 sieht es vor, dass der Rückschluss 32 Polschuhe 34 aufweist, die beispielsweise zwischen den Permanentmagneten 20, 21 und den Spulen 26, 27 sowie zwischen den beiden Spulen 26, 27 angeordnet sein können (Fig. 4). Diese Polschuhe 34 bewirken, dass das durch die Spulen 26, 27 generierte magnetische Feld besonders wirksam in Richtung des Läufers 28 gelenkt wird und die magnetische Flussdichte dort besonders hoch wird. Durch die Konzentration der magnetischen Flussdichte wirkt eine erhöhte Kraft auf den Läufer, wodurch dieser mit weniger Energieaufwand bewegbar ist.

Gleichermaßen vorteilhaft wirken sich Polschuhe 38 am Läufer 28 aus (Fig. 4). Auch hier bewirken die Polschuhe 38, dass das magnetische Feld der Permanentmagnete 20, 21 und der Spulen 26, 27 besonders effizient den Läufer 28 erfasst. Neben dem in der Fig. 4 dargestellten Ausführungsbeispiel des Läufers 28 sind noch weitere Ausführungsformen von Polschuhen 38 denkbar.

In der Fig. 5 wird die Schaltung der beiden Spulen 26, 27 stark simplifiziert dargestellt. Demnach sind die beiden sich gegenüberliegenden Enden der Spulen 26, 27 mit dem Erdpotenzial verbunden, wohingegen den jeweils anderen Enden der Spulen 26, 27 jeweils eine Diode 35, 36 zugeordnet sind. Die Dioden 35, 36 sind gegensinnig zueinander ausgerichtet, sodass deren Sperrrichtung ebenfalls unterschiedlich sind. Durch eine gepulste Bestromung der Spulen 26, 27 über die Dioden 35, 36, bzw. über eine elektrische Leitung 37, wird abwechselnd jeweils in einer der Spulen 26, 27 ein magnetisches Feld induziert, welches dem Feld der jeweiligen anderen Spule 26, 27 entgegen gerichtet ist.

Durch die hier dargestellte Anordnung der Permanentmagnete 20, 21 und der Spulen 26, 27 wird ein besonders geeignetes magnetisches Feld generiert, mit welchem sich der Läufer 28 in dem Gleitrohr 29 besonders einfach und verlässlich schalten lässt. Durch die Anordnung der besagten Elemente lassen sich herstellungsbedingte Abweichungen in der Dimensionierung der Permanentmagnete 20, 21, welche sich auf deren magnetische Felder auswirken, kompensieren, sodass diese Variationen keinen Einfluss haben auf das Schalten des Läufers 28 bzw. auf das Fokussieren der Optik.

### Bezugszeichenliste

- 10: Aktuator
- 11: Endoskop
- 12: Hauptkörper
- 13: Schaft
- 14: Griff
- 15: Anschluss
- 16: distales Ende
- 17: proximales Ende
- 18: Leiter
- 19: Stator
- 20: Permanentmagnet
- 21: Permanentmagnet
- 22: Endbereich
- 23: Endbereich
- 24: Nordpol
- 25: Südpol
- 26: Spule
- 27: Spule
- 28: Läufer
- 29: Gleitrohr
- 30: Anschlag
- 31: optische Achse
- 32: Rückschluss
- 33: Pfeilrichtung
- 34: Polschuh
- 35: Diode
- 36: Diode
- 37: elektrische Leitung
- 38: Polschuh

## Patentansprüche

1. Elektromagnetischer Aktuator (10) für ein chirurgisches oder medizinisches Instrument mit einem rohrförmigen Schaft (13), in dem ein zylindrischen Stator (19) angeordnet ist mit einem in dem Stator (19) beweglichen Element, wobei der Stator (19) mindestens zwei ringartige, axial magnetisierte Permanentmagnete (20, 21) und mindestens zwei Spulen (26, 27) zur Erzeugung eines elektromagnetischen Feldes aufweist und das bewegliche Element als ein in einem Gleitrohr (29) angeordneter Läufer (28) ausgebildet ist, wobei die Permanentmagnete (20, 21) derart um das Gleitrohr (29) angeordnet sind, dass ein magnetischer Südpol (25) des einen Permanentmagneten (20, 21) einem magnetischen Nordpol (24) des anderen Permanentmagnete (20, 21) zugewandt ist, **dadurch gekennzeichnet, dass** jeder Spule (26, 27) jeweils eine Diode (35, 36) vorgeschaltet ist, die unterschiedlich orientiert sind, so dass die Durchlassrichtungen der Dioden (35, 36) bezüglich der Spulen (26, 27) umgedreht sind und wobei die Spulen (26, 27) separat ansteuerbar eingerichtet sind, wobei die Spulen (26, 27) mit einem wechselnden Impuls elektromagnetischer Energie betreibbar eingerichtet sind.

2. Elektromagnetischer Aktuator (10) für ein chirurgisches oder medizinisches Instrument nach Anspruch 1, **dadurch gekennzeichnet, dass** die Spulen (26, 27) eine entgegengesetzte Wickelrichtung aufweisen.

3. Elektromagnetischer Aktuator (10) für ein chirurgisches oder medizinisches Instrument nach einem der vorangegangenen Ansprüche, **dadurch gekennzeichnet, dass** die Spulen (26, 27) zwischen den Permanentmagneten (20, 21) auf dem Gleitrohr (29) angeordnet sind, wobei die Permanentmagnete (20, 21) Endbereichen (22, 23) des Gleitrohres (29) zugeordnet sind.

4. Elektromagnetischer Aktuator (10) für ein chirurgisches oder medizinisches Instrument nach einem der vorangegangenen Ansprüche, **dadurch gekennzeichnet, dass** zwischen den Permanentmagneten (20, 21) ein zylindrischer Rückschluss (32) um die Spulen (26, 27) angeordnet ist, insbesondere dieser Rückschluss (32) Polschuhe (34) aufweist, die zwischen den Spulen (26, 27) und/oder zwischen den Permanentmagneten (20, 21) und den Spulen (26, 27) angeordnet sind.

5. Elektromagnetischer Aktuator (10) für ein chirurgisches oder medizinisches Instrument nach Anspruch 4, **dadurch gekennzeichnet, dass** die Spulen (26, 27) derart bestrombar sind, dass sich in dem Rückschluss (32) ein magnetischer Fluss ausbildet mit der gleichen Orientierung wie der magnetische Fluss der Permanentmagnete (20, 21).

6. Elektromagnetischer Aktuator (10) für ein chirurgisches oder medizinisches Instrument nach einem der vorangegangenen Ansprüche, **dadurch gekennzeichnet, dass** den Endbereichen (22, 23) des Gleitrohres (29) jeweils ein Anschlag (30) zugeordnet ist, um den Hub des beweglichen Läufers (28) zu begrenzen.

7. Elektromagnetischer Aktuator (10) für ein chirurgisches oder medizinisches Instrument nach einem der vorangegangenen Ansprüche, **dadurch gekennzeichnet, dass** der Läufer (28) als Hohlzylinder aus einem weichmagnetischen Material, vorzugsweise zur Aufnahme mindestens einer Linse, ausgebildet ist.

8. Elektromagnetischer Aktuator (10) für ein chirurgisches oder medizinisches Instrument nach einem der vorangegangenen Ansprüche, **dadurch gekennzeichnet, dass** der Läufer (28) mindestens zwei Polschuhe (38) aufweist, die den Endbereichen des Läufers (28) zugeordnet sind.

## Claims

1. An electromagnetic actuator (10) for a surgical or medical instrument comprising a tubular shaft (13), in which a cylindrical stator (19) is arranged with a movable element in the stator (19), wherein the stator (19) has at least two annular, axially magnetised permanent magnets (20, 21) and at least two coils (26, 27) for generating an electromagnetic field, and the movable element is formed as a rotor (28) arranged in a sliding tube (29), wherein the permanent magnets (20, 21) are arranged around the sliding tube (29) in such a way that a magnetic south pole (25) of the one permanent magnet (20, 21) faces a magnetic north pole (24) of the other permanent magnet (20, 21), **characterised in that** a diode (35, 36) is in each case connected upstream of each coil (26. 27), which are oriented differently such that the passage directions of the diodes (35, 36) are reversed with respect to the coils (26, 27), and wherein the coils (26, 27) are set up such that they can be activated separately, wherein the coils (26, 27) are set up such that they can be operated with an alternating pulse of electromagnetic energy.

2. The electromagnetic actuator (10) for a surgical or medical instrument according to claim 1, **characterised in that** the coils (26, 27) have an opposite winding direction.

3. The electromagnetic actuator (10) for a surgical or medical instrument according to any one of the preceding claims, **characterised in that** the coils (26, 27) are arranged between the permanent magnets (20, 21) on the sliding tube (29), wherein the permanent magnets (20, 21) are assigned to end regions (22, 23) of the sliding tube (29).

4. The electromagnetic actuator (10) for a surgical or medical instrument according to any one of the preceding claims, **characterised in that** a cylindrical back iron (32) is arranged around the coils (26, 27) between the permanent magnets (20, 21), this back iron (32) in particular has pole shoes (34), which are arranged between the coils (26, 27) and/or between the permanent magnets (20, 21) and the coils (26, 27).

5. The electromagnetic actuator (10) for a surgical or medical instrument according to claim 4, **characterised in that** the coils (26, 27) can be energised in such a way that a magnetic flux forms in in the back iron (32) with the same orientation as the magnetic flux of the permanent magnets (20, 21).

6. The electromagnetic actuator (10) for a surgical or medical instrument according to any one of the preceding claims, **characterised in that** a stop (30) is in each case assigned to the end regions (22, 23) of the sliding tube (29), in order to limit the stroke of the movable rotor (28).

7. The electromagnetic actuator (10) for a surgical or medical instrument according to any one of the preceding claims, **characterised in that** the rotor (28) is formed as hollow cylinder made of a soft magnetic material, preferably for receiving at least one lens.

8. The electromagnetic actuator (10) for a surgical or medical instrument according to any one of the preceding claims, **characterised in that** the rotor (28) has at least two pole shoes (38), which are assigned to the end regions of the rotor (28).

## Revendications

1. Actionneur électromagnétique (10) pour instrument chirurgical ou médical avec une gaine (13) de forme tubulaire dans laquelle est disposé un stator (19) cylindrique avec un élément mobile dans le stator (19), le stator (19) présentant au moins deux aimants permanents (20, 21) annulaires à aimantation axiale et au moins deux bobines (26, 27) pour la production d'un champ électromagnétique, et l'élément mobile étant conçu sous forme de curseur (28) placé dans un tube coulissant (29), les aimants permanents (20, 21) étant agencés autour du tube coulissant (29) de façon telle qu'un pôle magnétique sud (25) de l'un des aimants permanents (20, 21) fasse face à un pôle magnétique nord (24) de l'autre aimant permanent (20, 21), **caractérisé en ce qu'**une diode (35, 36) est respectivement montée en amont de chaque bobine (26, 27), ces diodes (35, 36) étant d'orientation différente de sorte que leurs sens passants par rapport aux bobines (26, 27) sont inversés, les bobines (26, 27) étant branchées de façon à pouvoir être commandées séparément et mises en service avec une impulsion alternée d'énergie électromagnétique.

2. Actionneur électromagnétique (10) pour instrument chirurgical ou médical selon la revendication 1, **caractérisé en ce que** les bobines (26, 27) ont un sens d'enroulement contraire.

3. Actionneur électromagnétique (10) pour instrument chirurgical ou médical selon l'une des revendications précédentes, **caractérisé en ce que** les bobines (26, 27) sont agencées entre les aimants permanents (20, 21) sur le tube coulissant (29), les aimants permanents (20, 21) étant affectés à des sections d'extrémité (22, 23) du tube coulissant (29).

4. Actionneur électromagnétique (10) pour instrument chirurgical ou médical selon l'une des revendications précédentes, **caractérisé en ce qu'**une culasse (32) cylindrique est agencée autour des bobines (26, 27) entre les aimants permanents (20, 21), cette culasse (32) présentant notamment des pièces polaires (34) agencées entre les bobines (26, 27) et/ou entre les aimants permanents (20, 21) et les bobines (26, 27).

5. Actionneur électromagnétique (10) pour instrument chirurgical ou médical selon la revendication 4, **caractérisé en ce que** les bobines (26, 27) peuvent être alimentées en courant de façon à ce qu'un flux magnétique de même orientation que le flux magnétique des aimants permanents (20, 21) se forme dans la culasse (32).

6. Actionneur électromagnétique (10) pour instrument chirurgical ou médical selon l'une des revendications précédentes, **caractérisé en ce qu'**une butée (30) est affectée à chaque section d'extrémité (22, 23) du tube coulissant (29) pour limiter la course du curseur (28) mobile.

7. Actionneur électromagnétique (10) pour instrument chirurgical ou médical selon l'une des revendications précédentes, **caractérisé en ce que** le curseur (28) est réalisé sous forme de cylindre creux en un matériau magnétique doux permettant de préférence d'y loger au moins une lentille.

8. Actionneur électromagnétique (10) pour instrument chirurgical ou médical selon l'une des revendications précédentes, **caractérisé en ce que** le curseur (28) présente au moins deux pièces polaires (38) affectées aux sections d'extrémité du curseur (28).
